(19) Europäisches Patentamt
European Patent Office
Office européen des brevets

(11) EP 0 659 719 B1

(12) # FASCICULE DE BREVET EUROPEEN

(45) Date de publication et mention de la délivrance du brevet:
**20.05.1998 Bulletin 1998/21**

(51) Int. Cl.[6]: **C07C 5/27**, B01J 21/12

(21) Numéro de dépôt: **94402765.5**

(22) Date de dépôt: **02.12.1994**

(54) **Procédé d'isomérisation squelettale des oléfines utilisant une matière à base d'alumine**

Verfahren zur Skelettisomerisierung von Olefinen in der ein auf Tonerde basiertes Material verwendet wird

Process for the skeletal isomerisation of olefins in presence of a material based on alumina

(84) Etats contractants désignés:
**BE DE ES FR GB NL**

(30) Priorité: **22.12.1993 FR 9315502**

(43) Date de publication de la demande:
**28.06.1995 Bulletin 1995/26**

(73) Titulaire:
**INSTITUT FRANCAIS DU PETROLE**
**92502 Rueil-Malmaison (FR)**

(72) Inventeurs:
- **Travers, Christine**
**F-92500 Rueil Malmaison (FR)**
- **Burzynski, Jean-Pierre**
**F-69110 Sainte-Foy-Les-Lyon (FR)**

(56) Documents cités:
**FR-A- 2 249 852**
**US-A- 4 038 337**
**US-A- 4 392 988**

Remarques:
Le dossier contient des informations techniques présentées postérieurement au dépôt de la demande et ne figurant pas dans le présent fascicule.

EP 0 659 719 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

### ART ANTERIEUR

La présente invention décrit un procédé d'isomérisation des oléfines présentant de 4 à 20 atomes de carbone et plus particulièrement d'isomérisation des n-butènes en isobutènes et des n-pentènes en isopentènes (iso amylènes), utilisant un catalyseur particulier.

La réduction des alkyls de plomb dans les essences a conduit le raffineur à envisager depuis quelques années, l'incorporation à l'essence de différents composés et en particulier, des alcools et des éthers, permettant d'augmenter l'indice d'octane. Outre le méthanol qui est l'un des plus intéressants additifs connu, le MTBE (méthyl-tertio-butyl éther), possède des propriétés antidétonnantes permettant l'amélioration de la qualité des essences et l'augmentation de leur indice d'octane, augmentation supérieure à celle obtenue avec le méthanol. Le MTBE possède également de nombreux autres avantages tels que :

- un point d'ébullition correspondant à celui des composants de l'essence qui présentent les plus faibles propriétés antidétonnantes,
- une tension de vapeur compatible avec les composants précités,
- un excellent point de congélation,
- une solubilité dans l'eau faible,
- une miscibilité complète avec les hydrocarbures etc...

Le MTBE est généralement obtenu à partir d'isobutène et de méthanol d'après la réaction suivante :

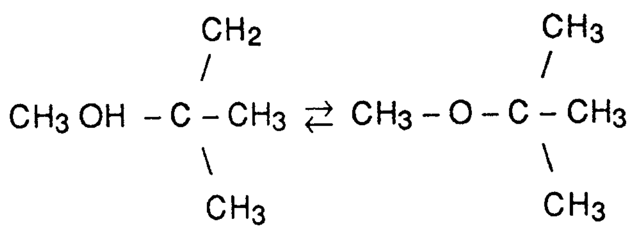

L'isobutène est généralement contenu dans les coupes oléfiniques $C_3$-$C_4$, issues des effluents du craquage catalytique, du vapocraquage, du craquage thermique et du procédé de viscoréduction. Cependant les quantités d'isobutène fournies par ces différents procédés ne sont pas suffisantes pour permettre un large développement du procédé de production de MTBE.

C'est pourquoi, il a été proposé, afin de produire de plus grandes quantités d'isobutène, d'isomériser complètement ou presque, les butènes contenus dans les effluents des procédés cités ci-dessus en isobutènes.

D'une façon similaire, pour fabriquer le TAME (tertio-amyl méthyl éther) il est recherché de plus grandes quantités d'isopentènes. L'invention propose un procédé d'isomérisation des n-pentènes en isopentènes.

De nombreux procédés associés à de nombreux catalyseurs ont été proposés dans la littérature. Les catalyseurs utilisés sont généralement à base d'alumine, ou plus particulièrement d'alumines activées ou traitées à la vapeur, qu'il s'agisse d'alumine éta ou gamma, d'alumines halogénées, de bauxite, d'alumines traitées avec des dérivés du bore, du silicium ou du zirconium et diverses silice-alumines etc...

Le brevet US 4,038,337 qui constitue l'art antérieur le plus proche, décrit un procédé d'isomérisation squelettale d'oléfines ayant au moins 4 atomes de carbone en présence d'un catalyseur à base d'alumine qui a été préalablement traitée par un composé du silicium de formule :

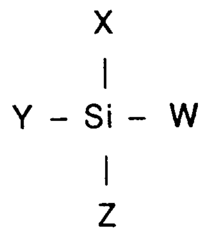

ou X, Y, Z, W sont choisis dans le groupe formé par R, OR, Cl, Br, $SiH_3$, COOR et $SiH_nCl_m$, R étant l'hydrogène ou un radical alkyl, cycloalkyl, aromatique, alkyl-aromatique ou alkyl-cycloaromatique, R ayant de 1 à 3 atomes de carbone, et n et m étant égaux à 1, 2 ou 3.

Pour l'imprégnation, le composé de silicium est utilisé tel que ou en solution organique.

La plupart de ces catalyseurs présente une conversion par passe relativement faible et une faible sélectivité due aux réactions parasites telles que le craquage et la polymérisation, ces dernières entraînant de plus une rapide décroissance des performances.

Le brevet US-A-4,392,988 décrit l'obtention d'une alumine à stabilités thermique et chimique améliorées, par traitement avec un polysiloxane. Aucune indication n'est donnée sur l'emploi de telle alumine dans le procédé d'isomérisation squelettale.

**OBJET DE L'INVENTION**

Il a maintenant été découvert qu'un catalyseur obtenu à partir d'une matière à base d'alumine préparée par un procédé dans lequel dans une première étape on traite une substance à base d'alumine avec une émulsion aqueuse d'au moins un polyorganosiloxane puis dans une seconde étape, on soumet le produit résultant à un traitement thermique, ledit catalyseur présente un rendement amélioré en isooléfine (isopentène par exemple), lors de l'isomérisation squelettale des oléfines (et du n-pentène par exemple). Ces catalyseurs montrent également d'excellentes propriétés mécaniques et/ou thermiques.

**DISCUSSION DÉTAILLÉE**

Dans le procédé suivant l'invention, il est possible d'isomériser une coupe $C_4$ oléfinique seule (après avoir enlevé la coupe $C_3$), la totalité de la coupe $C_3$-$C_4$ oléfinique, ou de préférence une coupe $C_5$ oléfinique ou plus généralement des hydrocarbures oléfiniques linéaires contenant de 4 à 20 atomes de carbone par molécule ou de préférence des hydrocarbures linéaires contenant de 5 à 20 atomes de carbone par molécule.

La charge à isomériser est mise au contact du catalyseur à une température comprise entre 300 °C et 570 °C (et de préférence entre 400 et 550 °C lorsqu'elle est constituée de butènes et/ou de pentènes) à une pression comprise entre 1 et 10 bars absolus (et préférentiellement entre 1 et 5 bars absolus lorsque la charge est constituée de butènes et/ou de pentènes).

La vitesse spatiale est comprise entre 0,1 et 10 $h^{-1}$ exprimée en volume de charge oléfinique par volume de catalyseur et par heure (et de manière préférée entre 0,5 et 6 $h^{-1}$ lorsque la charge est constituée de butènes et/ou de pentènes).

Le procédé peut être mis en oeuvre en présence d'eau, afin de minimiser les réactions secondaires indésirables ou en l'absence d'eau si ces réactions parasites ne sont pas suffisamment importantes (cas de la coupe $C_5$ ou plus généralement des coupes $C_5$-$C_{20}$). La quantité d'eau introduite dans le réacteur est telle que le rapport molaire $H_2O$/hydrocarbures oléfiniques soit compris entre 0 et 10 (et avantageusement entre 0,1 et 10, mieux entre 0,1 et 5 ou entre 0,1 et 3 et préférentiellement entre 0,5 et 3 lorsque la charge est constituée de butènes et/ou de pentènes).

Le catalyseur utilisé est une matière à base d'alumine préparée par un procédé en 2 étapes. Dans une première étape, on imprègne une substance à base d'alumine avec une émulsion aqueuse d'au moins un polyorganosiloxane et, dans une seconde étape, on fait subir au produit résultant un traitement thermique.

Selon une première variante de l'invention, le procédé est caractérisé en ce que, dans la première étape, on imprègne une matière à base d'alumine avec une émulsion aqueuse d'au moins un polyorganosiloxane et, dans la seconde étape, on fait subir un traitement thermique à la matière ainsi imprégnée.

L'imprégnation est de préférence réalisée à sec, c'est-à-dire que le volume total de l'émulsion aqueuse utilisée est approximativement égal au volume poreux total présenté par la matière à imprégner; il peut ainsi être nécessaire d'effectuer un ajout d'eau à l'émulsion afin d'obtenir le volume d'imprégnation requis.

En général, la matière à base d'alumine utilisée contient au moins 50 %, de préférence au moins 70 % en poids d'alumine. De préférence, cette matière est de l'alumine seule.

Cette matière de départ peut avoir été mise en forme avant la première étape et peut se présenter sous forme de billes, d'agglomérés, de granulés d'extrudés, de pastilles ou de concassés : elle se présente de préférence sous forme de billes ou d'extrudés.

On peut employer une matière à base de billes d'alumine fabriquées par coagulation en gouttes (méthode "oil-drop") dont la teneur en métaux alcalins est inférieure à 100 ppm et qui présente de préférence une grande résistance à l'attrition. Une telle alumine peut être préparée selon l'enseignement des brevets GB-B-2134536, EP-B-15801 ou EP-B-97539.

La surface spécifique de la matière de départ, par exemple de l'alumine, peut avantageusement être comprise entre 10 et 450 $m^2/g$, en particulier entre 60 et 350 $m^2/g$, son volume poreux étant par exemple compris entre 0,3 et 1,2 $cm^3/g$.

Selon une seconde variante de l'invention, le procédé est caractérisé en ce que, dans la première étape, on introduit une émulsion aqueuse d'au moins un polyorganosiloxane lors de la mise en forme d'une matière à base d'alumine et, dans la seconde étape, on fait subir un traitement thermique à la matière ainsi mise en forme, qui contient le polyorganosiloxane.

Dans cette seconde variante de l'invention, la mise en forme de la matière à base d'alumine peut s'effectuer par toute technique bien connue de l'homme du métier, notamment par malaxage-extrusion, par coagulation en gouttes (méthode "oil-drop") ou par granulation (dragéification).

Ainsi, la première étape du procédé selon la seconde variante de l'invention peut par exemple comprendre, suivant le traitement de mise en forme utilisé :

l'introduction de l'émulsion aqueuse d'au moins un polyorganosiloxane au cours du malaxage d'un gel d'alumine et d'un agent peptisant (tel que l'acide nitrique, l'acide acétique, l'urée) et éventuellement d'eau, suivie d'une extrusion, de manière à obtenir des extrudés; ou
l'introduction de l'émulsion aqueuse d'au moins un polyorganosiloxane dans une suspension ou une dispersion d'alumine avant égouttage dans une colonne de coagulation en gouttes, de manière à obtenir des billes; ou
l'introduction de l'émulsion aqueuse d'au moins un polyorganosiloxane par aspersion de ladite émulsion et de poudre d'alumine au cours de la granulation(ou dragéification) en assiette tournante, de manière à obtenir des granulés de préférence sensiblement spériques.

Selon la première étape du procédé de l'invention, on traite une substance à base d'alumine avec une émulsion aqueuse d'au moins un polyorganosiloxane, de préférence linéaire ou cyclique. Ce polyorganosiloxane est, de manière avantageuse, sensiblement insoluble dans l'eau.

De préférence, le polyorganosiloxane utilisé est une huile polyorganosiloxanique.

Ce polyorganosiloxane peut ainsi être représenté par la formule (1) suivante :

$$R^1\ R^2{}_2Si\ O(Si\ R^2{}_2O)_n\ Si\ R^2{}_2\ R^1 \qquad (1)$$

dans laquelle :

- les radicaux $R^1$ sont identiques ou différents et représentent
  - un radical phényle, alkyle ou alcényle linéaire ou ramifié pouvant contenir jusqu'à 6 atomes de carbone, éventuellement substitué par au moins un groupe cyano,
  - un radical hydroxy,
  - un radical alcoxy en $C_1$-$C_4$, ou
  - un radical acyloxy en $C_1$-$C_4$,
- les radicaux $R^2$ sont identiques ou différents et représentent un radical phényle, alkyle ou alcényle linéaire ou ramifié pouvant contenir jusqu'à 6 atomes de carbone, éventuellement substitué par au moins un groupe cyano,
- n est un nombre supérieur ou égal à 1.

Généralement, au moins 50%, de préférence au moins 80%, en nombre de l'ensemble des radicaux $R^1$ et $R^2$ représentant des radicaux méthyle. Les radicaux alkyle ou alcényle les plus courants sont les radicaux méthyle, éthyle, propyle et vinyle.

De préférence, on emploie un composé de formule (1) dans laquelle les radicaux $R^2$ sont identiques; ledit composé est alors un polydiorganosiloxane.

En particulier, le polydiorganosiloxane utilisé dans la présente invention est de préférence un polydiorganosiloxane de formule (1) dans laquelle tous les radicaux $R^2$ sont des radicaux méthyle, les radicaux $R^1$ étant alors habituellement identiques.

Le polyorganosiloxane utilisé dans la présente invention peut ainsi être par exemple un polydiorganosiloxane de formule (1) dans laquelle tous les radicaux $R^1$ sont des radicaux hydroxy (HO-) et tous les radicaux $R^2$ sont des radicaux méthyle : le composé de formule (1) est alors un (bishydroxy)polydiméthylsiloxane. De préférence, n est dans ce cas égal à 1.

Le polyorganosiloxane utilisé dans la présente invention peut ainsi être par exemple un polydiorganosiloxane de formule (1) dans laquelle tous les radicaux $R^1$ et $R^2$ sont des radicaux méthyle : le composé de formule (1) est alors un polydiméthylsiloxane. De préférence, n est dans ce cas égal à 1.

Le polyorganosiloxane présente une viscosité habituellement inférieure à 50 000 mPa.s à 25°C, en général comprise entre 50 et 40 000 mPa.s à 25°C, de préférence entre 100 et 10 000 mPa.s à 25°C.

De manière préférée, on utilise un polydiméthylsiloxane linéaire présentant par exemple une viscosité comprise entre 100 et 1 000 mPa.s à 25°C.

Le polyorganosiloxane employé peut être un polyorganosiloxane cyclique, dans lequel les radicaux sont par exemple identiques et, en particulier, sont tous des radicaux méthyle : on peut citer notamment l'héxaméthylcyclotrisiloxane et l'octaméthylcyclotétrasiloxane.

En général, l'émulsion aqueuse d'au moins un polyorganosiloxane est du type huile dans eau et est anionique ou, de préférence, non-ionique; elle peut être stabilisée par un agent tensio-actif anionique ou, de préférence, non-ionique.

Ce type d'émulsion est bien connue : elle peut être préparée directement par polymérisation du monomère correspondant en émulsion selon un procédé décrit notamment dans les brevets US-A-2891920 et US-A-3294725; de même, elle peut être obtenue par mise en émulsion aqueuse du polymère en stabilisant l'émulsion à l'aide d'un agent tensioactif selon un procédé décrit notamment dans les demandesde brevets FR-A-2064563, FR-A-2094322, FR-A-2114230 et EP-A-169098.

A titre d'agents tensio-actifs anioniques, on peut citer les sels de métaux alcalins d'acide gras, d'alkylphosphate, d'alkylsulfates, d'alkylsulfonates, d'arylsulfonates, d'alkyarylsulfonates, de sulfosuccinate.

A titre d'agents tensio-actifs non-ioniques, on peut citer les alcools gras polyéthoxylés, les alkylphénolpolyéthoxylés, les acides gras polyéthoxylés. Cette émulsion présente, en général, une teneur en extrait sec inférieure à 50% en poids.

On met généralement en oeuvre dans le procédé de l'invention une quantité de polyorganosiloxane telle que la quantité de silice introduite dans la matière à base d'alumine est comprise entre 0,05 et 15 %, de préférence entre 0,5 et 5 % , en poids de silice par rapport au produit fini, c'est-à-dire à la matière à base d'alumine obtenue à l'issue dudit procédé.

Selon une variante du procédé de l'invention, on peut introduire dans la matière de départ avant, après ou simultanément au polysiloxane au moins un élément métallique de la classification périodique des éléments. A titre d'exemples non limitatifs de tels éléments métalliques, on peut citer le platine, le palladium, le nickel, le cuivre, le manganèse, le molyodène, l'argent, le chrome et de façon préférée le titane et le bore. La matière à base d'alumine est alors un support de catalyseur; et le catalyseur comprend au moins le support et le(s) élément(s) métallique(s).

Selon la seconde étape du procédé de l'invention, on fait subir à la matière imprégnée un traitement thermique. Ce traitement thermique est généralement effectué à une température comprise entre 70 et 1200 °C.

Ce traitement thermique consiste de préférence en un séchage suivi d'une calcination.

Le séchage est alors habituellement réalisé à une température comprise entre 70 et 450 °C, par exemple entre 80 et 400 °C ; la durée de séchage varie en général entre 0,5 et 6 heures.

La calcination est alors habituellement réalisée à une température comprise entre 450 et 1200 °C, par exemple entre 500 et 900 °C ; la durée de la calcination varie en général entre 1 et 24 heures.

Les autres conditions du séchage et de la calcination sont celles qui sont généralement employées par l'homme du métier.

L'invention est illustrée par les exemples suivants.

Les performances en isomérisation seront exprimées, par exemple dans le cas des butènes, par :

1/ <u>la conversion des butènes</u>

$$C = \frac{\Sigma\ (\%\ n\ \text{butènes})\ \text{charge} - \Sigma\ (\%\ n\ \text{butènes})\ \text{effluent}}{\Sigma\ (\%\ n\text{-butènes})\ \text{charge}} \times 100$$

2/ <u>La sélectivité en isobutène</u>

$$S = \frac{(\%\ \text{isobutène})\ \text{effluent} - (\%\ \text{isobutène})\ \text{charge}}{\Sigma\ (\%\ \text{n-butènes})\ \text{charge} - \Sigma\ (\%\ \text{n butènes})\ \text{effluent}} \times 100$$

3/ <u>Le rendement en isobutène</u>

$$R = C \times S / 100$$

Dans le cas où la charge est composé de pentènes, les performances en isomérisation seront exprimées par

1/ <u>la conversion des n-pentènes</u>

$$C = \frac{\Sigma\ (\%\ \text{n-pentènes})\ \text{charge} - \Sigma\ (\%\ \text{n-pentènes})\ \text{effluent}}{\Sigma\ (\%\ \text{n-pentènes})\ \text{charge}}\ X\ 100$$

2/ <u>La sélectivité en isopentènes "tamables" *</u>

$$S = \frac{\Sigma\ (\%\ 2MC_{4}{}^{1} + \%\ 2MC_{4}{}^{2})_{\text{effluent}} - \Sigma\ (\%\ 2MC_{4}{}^{1} + \%\ 2MC_{4}{}^{2})_{\text{charge}}}{\Sigma\ (\%\ \text{n-pentènes})\ \text{charge} - \Sigma\ (\%\ \text{n-pentènes}}\ X\ 100$$

3/ <u>Le rendement en isopentènes</u>

$$R = C\ X\ S/100$$

Les exemples qui suivent, précisent l'invention sans en limiter la portée.

**Exemple 1 - Catalyseur A conforme à l'invention**

L'émulsion aqueuse de polyorganosiloxane utilisé dans ces exemples est le RHODORSIL® EMULSION E 1 P qui est une émulsion aqueuse nonionique d'huile polydiméthylsiloxanique commercialisée par la société Rhône Poulenc Chimie et qui présente une viscosité du fluide de base (huile polydiméthylsiloxanique) d'environ 350 mPa.s à 25°C et une teneur en extrait sec de 35% en poids environ.

On imprègne 100 grammes d'extrudés d'alumine, préparés par malaxage-extrusion de gel d'alumine suivi d'un séchage et d'une calcination, par une solution obtenue en diluant 12 grammes de RHODORSIL® EMULSION E 1 P avec de l'eau jusqu'à un volume total d'imprégnation de 60 $cm^3$.

Le support imprégné est, ensuite, séché 1 heure à 140 °C puis calciné à 600 °C pendant 2 heures.

On obtient, ainsi, des extrudés d'alumine contenant 2,85 % de $SiO_2$.

Ce catalyseur est mis en oeuvre en isomérisation d'une coupe $C_4$ oléfinique dont la composition figure tableau 1. Les conditions opératoires sont les suivantes :

- LHSV = 2 $h^{-1}$ (volume de charge oléfinique/volume de catalyseur $x^h$)
- $H_2O/C_4$= (mole) = 2
- T = 530 °C
- p = 1,5 bars absolus

Les performances obtenues figurent tableau 1 après 1 heure de fonctionnement.

**EXEMPLE 2 - Catalyseur B conforme à l'invention**

On imprègne 100 grammes de billes d'alumine, préparées par coagulation de goutte d'une suspension d'alumine ou par granulation de poudre d'alumine, par une solution obtenue en diluant 12 grammes de RHODORSIL® EMUL-

*tamables : isomères qui rèagissent avec le mèthanol pour donner du TAME (tertio amyl mèthyl èther)
*2 $MC_4$ 1 = 2 mèthyl butène 1
*2 $MC_4$ 2 = 2 mèthyl butène 2

SION E 1 P avec de l'eau jusqu'à un volume total d'imprégnation de 60 $cm^3$.

Le support imprégné obtenu est, ensuite, séché à 140 °C pendant 1 heure puis calciné sous air à 600 °C, 2 heures.

On obtient ainsi des billes d'alumine contenant 2,9 % de $SiO_2$.

Le catalyseur B est mis en oeuvre dans le procédé d'isomérisation d'une coupe $C_4$ oléfinique, dans les conditions opératoires décrites ci-dessus.

Les performances obtenues figurent tableau 1 après 1 heure de fonctionnement.

**EXEMPLE 3**

Les catalyseurs A et B décrits respectivement dans les exemples 1 et 2 sont mis en oeuvre en isomérisation du n-pentènes dans les conditions suivantes :

LHSV = 1$h^{-1}$
$H_2O/C_5$ (mole) = 0
T = 410°C
P = 1 bar.

Les performances obtenues figurent Tableau 2 après 1 heure de fonctionnement.

**EXEMPLE 4**

Les catalyseurs A et B décrits respectivement dans les exemples 1 et 2 sont mis en oeuvre en isomérisation du n-pentènes dans les conditions suivantes :

LHSV = 1$h^{-1}$
$H_2O/C_5$ (mole) = 1
T = 410°C
P = 1 bar.

Les performances obtenues figurent Tableau 3 après 1 heure de fonctionnement.

Tableau 1

| $H_2O/C_4$= (mole) | - | 2 | 2 |
|---|---|---|---|
| LHSV (h-1) | - | 2 | 2 |
| Temps de fonctionnement (h) | - | 1 | 1 |
| **% Poids** | **Charge** | **Catalyseur A** | **Catalyseur B** |
| $CH_4$ | 0 | 0,077 | 0,066 |
| $C_2$ | 0 | 0,019 | 0,018 |
| $C_2^=$ | 0 | 0,143 | 0,138 |
| $C_3$ | 0 | 0,115 | 0,113 |
| $C_3^=$ | 0 | 2,085 | 2,116 |
| $iC_4$ | 0,424 | 0,526 | 0,523 |
| $nC_4$ | 16,662 | 16,716 | 16,702 |
| $C_4^=$ 2TR | 2,046 | 21,121 | 21,224 |
| $C_4^=$ 1 | 70,59 | 17,342 | 16,802 |
| $iC_4^=$ | 6,66 | 23,955 | 24,298 |
| $C_4^=$ 2Cis | 2,744 | 16,385 | 16,335 |
| $C_4^{==}$ | 0,037 | 0,08 | 0,074 |
| $C_5^+$ | 0,095 | 1,414 | 1,57 |
| Conversion % | - | 27,23 | 27,86 |
| Sélectivité % | - | 84,23 | 83,98 |
| Rendement % | | 22,94 | 23,40 |

Tableau 2

| | Catalyseur A | Catalyseur B |
|---|---|---|
| Conversion (%) | 70,0 | 70,2 |
| Sélectivité (%) | 75,0 | 74,8 |
| Rendement | 52,5 | 52,5 |

Tableau 3

| | Catalyseur A | Catalyseur B |
|---|---|---|
| Conversion (%) | 63,0 | 62,5 |
| Sélectivité (%) | 80,0 | 80,2 |
| Rendement | 50,4 | 50,1 |

**Revendications**

**1.** Procédé d'isomérisation squelettale d'oléfines renfermant de 4 à 20 atomes de carbone, par passage de la charge

contenant les oléfines sur un catalyseur obtenu à partir d'une matière à base d'alumine, procédé caractérisé en ce qu'il se déroule à une température comprise entre 300 et 570°C, une pression comprise entre 1 et 10 bars, une vitesse spatiale (volume de charge oléfinique par volume de catalyseur et par heure) comprise entre 0,1 et 10 $h^{-1}$, et lorsque la charge est une coupe $C_4$ ou $C_3$-$C_4$, en présence d'eau en quantité telle que le rapport molaire eau/hydrocarbures oléfiniques soit d'au plus 10 et lorsque la charge est l'une des coupes $C_5$ à $C_{20}$, le procédé se déroule en l'absence ou en présence d'eau en quantité telle que le rapport molaire eau/hydrocarbures soit d'au plus 10, et en ce que la matière à base d'alumine est obtenue par un procédé dans lequel dans une première étape, on traite une substance à base d'alumine avec une émulsion aqueuse d'au moins un polyorganosiloxane, et, dans une seconde étape, on soumet le produit résultant à un traitement thermique.

**2.** Procédé selon la revendication 1, caractérisé en ce que la matière à base d'alumine contient 0,05 à 15% de silice.

**3.** Procédé selon l'une des revendications précédentes caractérisé en ce que dans la première étape, on imprègne une matière à base d'alumine avec une émulsion aqueuse d'au moins un polyorganosiloxane et, dans la seconde étape, on fait subir un traitement thermique à la matière ainsi imprégnée.

**4.** Procédé selon l'une des revendications précédentes caractérisé en ce que ladite matière de départ a été mise en forme préalablement à la première étape.

**5.** Procédé selon l'une des revendications 1 ou 2, caractérisé en ce que, dans la première étape, on introduit une émulsion aqueuse d'au moins un polyorganosiloxane lors de la mise en forme d'une matière à base d'alumine et, dans la seconde étape, on fait subir un traitement thermique à la matière ainsi mise en forme.

**6.** Procédé selon la revendication 5 caractérisé en ce que ladite mise en forme s'effectue par malaxage-extrusion, par coagulation en gouttes ou par granulation.

**7.** Procédé selon l'une des revendications précédentes caractérisé en ce que ledit polyorganosiloxane est une huile polyorganosiloxanique.

**8.** Procédé selon l'une des revendications précédentes caractérisé en ce que ledit polyorganosiloxane est représenté par la formule (1) suivante:

$$R^1 R^2_2 Si O (Si R^2_2 O)_n Si R^2_2 R^1 \quad (1)$$

dans laquelle :

- les radicaux $R^1$ sont identiques ou différents et représentent
  - o un radical phényle, alkyle ou alcényle linéaire ou ramifié pouvant contenir jusqu'à 6 atomes, éventuellement substitué par au moins un groupe cyano,
  - o un radical hydroxy
  - o un radical alcoxy en $C_1$-$C_4$, ou
  - o un radical acyloxy en $C_1$-$C_4$,
- les radicaux $R^2$ sont identiques ou différents et représentent un radical phényle, alkyle ou alcényle linéaire ou ramifié, éventuellement substitué par au moins un groupe cyano,
- n est un nombre supérieur ou égal à 1.

**9.** Procédé selon la revendication 8 caractérisé en ce que ledit polyorganosiloxane est représenté par la formule (1) dans laquelle au moins 50%, de préférence au moins 80%, en nombre de l'ensemble des radicaux $R^1$ et $R^2$ sont des radicaux méthyle.

**10.** Procédé selon l'une des revendications 8 et 9 caractérisé en ce que les radicaux $R^1$ sont identiques et représentent des radicaux hydroxy ou méthyle et les radicaux $R^2$ sont des radicaux méthyle.

**11.** Procédé selon la revendication 10 caractérisé en ce que n est égal à 1.

**12.** Procédé selon l'une des revendications précédentes caractérisé en ce que ledit polyorganosiloxane est un polydiméthylsiloxane linéaire présentant une viscosité inférieure à 50 000 mPa.s à 25°C.

**13.** Procédé selon l'une des revendications précédentes caractérisé en ce que ledit polyorganosiloxane est un polydiorganosiloxane cyclique, dans lequel les radicaux sont tous des radicaux méthyle.

**14.** Procédé selon l'une des revendications précédentes caractérisé en ce que ladite émulsion aqueuse est non-ionique.

**15.** Procédé selon l'une des revendications précédentes caractérisé en ce que la quantité de polyorganosiloxane utilisée est telle que la matière à base d'alumine obtenue contient entre 0,5 et 5 % en poids de silice.

**16.** Procédé selon l'une des revendications précédentes caractérisé en ce que, dans la seconde étape, ledit traitement thermique est effectué à une température comprise entre 70 et 1200 °C.

**17.** Procédé selon l'une des revendications précédentes caractérisé en ce que, dans la seconde étape, ledit traitement thermique consiste en un séchage suivi d'une calcination.

**18.** Procédé selon l'une des revendications précédentes caractérisé en ce que ladite matière obtenue constitue le catalyseur à base d'alumine.

**19.** Procédé selon l'une des revendications précédentes caractérisé en ce que la matière obtenue constitue le support de catalyseur, ledit catalyseur contenant également au moins un élément métallique de la classification périodique et/ou du bore.

**20.** Procédé selon l'une des revendications précédentes caractérisé en ce que la matière obtenue constitue le support de catalyseur, ledit catalyseur contenant également au moins un élément choisi dans le groupe formé par le platine, le palladium, le nickel, le cuivre, le manganèse, le molybdène, l'argent, le chrome.

**21.** Procédé selon l'une des revendications précédentes caractérisé en ce que la matière obtenue constitue le support de catalyseur, ledit catalyseur contenant également au moins un élément choisi dans le groupe formé par le titane et le bore.

**22.** Procédé selon l'une des revendications précédentes caractérisé en ce que la charge est une coupe $C_5$ oléfinique.

**23.** Procédé selon l'une des revendications précédentes caractérisé en ce que le rapport molaire eau/hydrocarbures oléfiniques est compris entre 0,1 et 3.

**24.** Procédé selon la revendication 22, caractérisé en ce qu'il se déroule en l'absence d'eau.

## Claims

**1.** A skeletal isomerisation process for olefins containing 4 to 20 carbon atoms, comprising passing a feedstock containing the olefins over a catalyst produced from an alumina-based compound, characterised in that it is carried out at a temperature of between 300°C and 570°C, a pressure of between 1 and 10 bars, a space velocity (volume of olefin feedstock per volume of catalyst per hour) of between 0.1 and 10 $h^{-1}$ and, if the feedstock is a $C_4$ or $C_3$-$C_4$ cut, in the presence of a quantity of water such that the water/olefinic hydrocarbon molar ratio is no more than 10, and if the feedstock is one of the $C_5$-$C_{20}$ cuts, the process is carried out without water or with water in a quantity such that the water/olefinic hydrocarbon molar ratio is no more than 10, and in that the alumina-based compound is obtained by a process in wich, in a first step, an alumina-based substance is treated with an aqueous emulsion of at least one polyorganosiloxane, the resulting product being heat treated in a second step.

**2.** Process according to claim 1, characterised in that the alumina-based compound contains 0.50 % to 15 % of silica.

**3.** Process according to claim 1 or claim 2, characterised in that, in the first step, an alumina-based compound is impregnated with an aqueous emulsion of at least one polyorganosiloxane the impregnated compound being heat treated in the second step.

**4.** Process according to any one of the preceding claims, characterised in that said starting compound is formed prior

to the first step.

5. Process according to claim 1 or claim 2, characterised in that, in the first step, an aqueous emulsion of at least one polyorganosiloxane is introduced during forming of the alumina-based compound the formed compound being heat treated in the second step.

6. Process according to claim 5, characterised in that said forming is carried out by mixing-extrusion, drop coagulation or granulation.

7. Process according to any one of the preceding claims, characterised in that said polyorganosiloxane is a polyorganosiloxane oil.

8. Process according to any one of the preceding claims, characterised in that said polyorganosiloxane has the following formula (1):

$$R^1R^2_2SiO(SiR^2_2O)_nSiR^2_2R^1 \quad (1)$$

wherein:

- radicals $R^1$ may be identical or different and represent
  - a linear or branched phenyl, alkyl or alkenyl radical containing up to 6 carbon atoms, which may be substituted by at least one cyano group,
  - a hydroxy radical,
  - a $C_1$-$C_4$ alkoxy radical, or
  - a $C_1$-$C_4$ acyloxy radical,
- radicals $R^2$ may be identical or different and represent a linear or branched phenyl, alkyl or alkenyl radical which may be substituted by a cyano radical,
- n is greater than or equal to 1.

9. Process according to claim 8, characterised in that said polyorganosiloxane has formula (1) wherein at least 50%, preferably at least 80%, by number of the ensemble of radicals $R^1$ and $R^2$ represent methyl radicals.

10. Process according to claim 8 or claim 9, characterised in that radicals $R^1$ are identical and represent hydroxy or methyl radicals, and radicals $R^2$ are methyl radicals.

11. Process according to claim 10, characterised in that n equals 1.

12. Process according to any one of the preceding claims, characterised in that said polyorganosiloxane is a linear polydimethylsiloyane with a viscosity of less than 50 000 mPa.s at 25°C.

13. Process according to any one of the preceding claims, characterised in that said polyorganosiloxane is a cyclic polydiorganosiloxane, wherein all the radicals are methyl radicals.

14. Process according to any one of the preceding claims, characterised in that said aqueous emulsion is non ionic.

15. Process according to any one of the preceding claims, characterised in that the quantity of polyorganosiloxane used is such that the alumina-based compound produced contains between 0.5% and 5% by weight of silica.

16. Process according to any one of the preceding claims, characterised in that, in the second step, said heat treatment is carried out at a temperature of between 70°C and 1200°C.

17. Process according to any one of the preceding claims, characterised in that, in the second step, said heat treatment consists of drying followed by calcining.

18. Process according to any one of the preceding claims, characterised in that said compound obtained constitutes the alumina-based catalyst.

19. Process according to any one of the preceding claims, characterised in that said compound obtained constitutes the catalyst support, said catalyst also containing at least one metallic element from the periodic classification and/or boron.

20. Process according to any one of the preceding claims, characterised in that said compound obtained constitutes the catalyst support, said catalyst also containing at least one element selected from the group formed by platinum, palladium, nickel, copper, manganese, molybdenum, silver and chromium.

21. Process according to any one of the preceding claims, characterised in that said compound obtained constitutes the catalyst support, said catalyst also containing at least one element selected from the group formed by titanium and boron.

22. Process according to any one of the preceding claims, characterised in that said feedstock is a $C_5$ olefin cut.

23. Process according to any one of the preceding claims, characterised in that said water/olefinic hydrocarbon molar ratio is between 0.1 and 3.

24. Process according to claim 22, characterised in that it is carried out in the absence of water.

**Patentansprüche**

1. Verfahren zur Skelettisomerisierung von 4 bis 20 Kohlenstoffatome enthaltenden Olefinen durch Durchgang der die Olefine enthaltenden Charge auf einem aus einem Material auf Aluminiumoxidgrundlage erhaltenen Katalysator, dadurch gekennzeichnet, daß es abläuft bei einer Temperatur zwischen 300 und 570°C, einem Druck zwischen 1 und 10 bar, einer Volumengeschwindigkeit (Volumen der olefincharge pro Volumen des Katalysators und pro Stunde) zwischen 0,1 und 10 $h^{-1}$, und wenn die Charge eine $C_4$- bder $C_3$-$C_4$-Charge ist, in Gegenwart von Wasser in solcher Menge, daß das molare Verhältnis Wasser/olefinische Kohlenwasserstoffe höchstens 10 ist, und wenn die Charge eine der $C_5$- bis $C_{20}$-Chargen ist, in Abwesenheit oder in Gegenwart von Wasser in solcher Menge, daß das molare Verhältnis Wasser/Kohlenwasserstoffe höchstens 10 ist, und daß das Material auf Aluminiumoxidgrundlage durch ein Verfahren erhalten wird, bei dem in einem ersten Schritt eine Substanz auf Aluminiumoxidgrundlage mit einer wäßrigen Emulsion wenigstens eines Polyorganosiloxans behandelt und in einem zweiten Schritt das resultierende Erzeugnis einer Wärmebehandlung unterworfen wird.

2. Verfahren nach Anspruch 1, dadurch gekennzeichnet, daß das Material auf Aluminiumoxidgrundlage 0,05 bis 15 % Siliciumdioxid enthält.

3. Verfahren nach einem der vorhergehenden Ansprüche, dadurch gekennzeichnet, daß im ersten Schritt ein Material auf Aluminiumoxidgrundlage mit einer wäßrigen Lösung wenigstens eines Polyorganosiloxans imprägniert und im zweiten Schritt das so imprägnierte Material einer Wärmebehandlung unterzogen wird.

4. Verfahren nach einem der vorhergehenden Ansprüche, dadurch gekennzeichnet, daß das Ausgangsmaterial vor dem ersten Schritt geformt worden ist.

5. Verfahren nach einem der Ansprüche 1 oder 2, dadurch gekennzeichnet, daß im ersten Schritt eine wäßrige Lösung wenigstens eines Polyorganosiloxans bei der Formung eines Materials auf Aluminiumoxidgrundlage eingeführt wird, und daß im zweiten Schritt das so geformte Material einer Wärmebehandlung unterzogen wird.

6. Verfahren nach Anspruch 5, dadurch gekennzeichnet, daß die Formung durch Knetextrusion, durch Tropfen-Coagulation bder durch Granulierung erfolgt.

7. Verfahren nach einem der vorhergehenden Ansprüche, dadurch gekennzeichnet, daß das Polyorganosiloxan ein Polyorganosiloxanöl ist.

8. Verfahren nach einem der vorhergehenden Ansprüche, dadurch gekennzeichnet, daß Polyorganosiloxan durch folgende Formel (1) gegeben ist:

$$R^1 R^2_2 Si O (Si R^2_2 O)_n Si R^2_2 R^1 \quad (1),$$

wobei

- die Radikale $R^1$ identisch oder verschieden sind und
  - o ein Phenyl-, Alkyl- oder lineares oder verzweigtes Alkenylradikal mit bis zu 6 Atomen, gegebenenfalls mit wenigstens einer Zyanogruppe substituiert,
  - o ein Hydroxyradikal,
  - o ein $C_1$-$C_4$-Alkoxyradikal, oder
  - o ein $C_1$-$C_4$-Acyloxyradikal darstellen,
- die Radikale $R^2$ identisch oder verschieden sind und ein Phenyl-Alkyl- oder lineares oder verzweigtes Alkenylradikal, gegebenenfalls mit einer Cyanogruppe substituiert, darstellen,
- n eine Zahl größer oder gleich 1 ist.

**9.** Verfahren nach Anspruch 8, dadurch gekennzeichnet, daß das Polyorganosiloxan durch die Formel (1) gegeben ist, in der mindestens 50 %, vorzugsweise mindestens 80% der Anzahl der $R^1$- und $R^2$-Radikale Methylradikale sind.

**10.** Verfahren nach einem der Ansprüche 8 und 9, dadurch gekennzeichnet, daß die Radikale $R^1$ identisch sind und Hydroxy- oder Methylradikale darstellen und die Radikale $R^2$ Methylradikale sind.

**11.** Verfahren nach Anspruch 10, dadurch gekennzeichnet, daß n gleich 1 ist.

**12.** Verfahren nach einem der vorhergehenden Ansprüche, dadurch gekennzeichnet, daß das Polyorganosiloxan ein lineares Polydimethylsiloxan mit einer Viskosität unterhalb 50 000 mPa.s bei 25°C ist.

**13.** Verfahren nach einem der vorhergehenden Ansprüche, dadurch gekennzeichnet, daß das Polyorganosiloxan ein zyklisches Polydiorganosiloxan ist, bei dem die Radikale alle Methylradikale sind.

**14.** Verfahren nach einem der vorhergehenden Ansprüche, dadurch gekennzeichnet, daß die wäßrige Emulsion nichtionisch ist.

**15.** Verfahren nach einem der vorhergehenden Ansprüche, dadurch gekennzeichnet, daß die verwendete Menge an Polyorganosiloxan derart ist, daß das erhaltene Material auf Aluminiumoxidgrundlage zwischen 0,5 und 5 Gew.-% Siliciumdioxid enthält.

**16.** Verfahren nach einem der vorhergehenden Ansprüche, dadurch gekennzeichnet, daß im zweiten Schritt die Wärmebehandlung bei einer Temperatur zwischen 70 und 1200°C durchgeführt wird.

**17.** Verfahren nach einem der vorhergehenden Ansprüche, dadurch gekennzeichnet, daß im zweiten Schritt die Wärmebehandlung aus einer Trocknung, gefolgt von einer Kalzinierung besteht.

**18.** Verfahren nach einem der vorhergehenden Ansprüche, dadurch gekennzeichnet, daß das erhaltene Material den Katalysator auf Aluminiumoxidgrundlage bildet.

**19.** Verfahren nach einem der vorhergehenden Ansprüche, dadurch gekennzeichnet, daß das erhaltene Material den Katalysatorträger bildet, wobei der Katalysator gleichfalls ein metallisches Element des Periodensystems und/oder Bor enthält.

**20.** Verfahren nach einem der vorhergehenden Ansprüche, dadurch gekennzeichnet, daß das erhaltene Material den Katalysatorträger bildet, wobei der Katalysator ferner wenigstens ein Element, ausgewählt aus der durch Platin, Palladium, Nickel, Kupfer, Mangan, Molybden, Silber, Chrom gebildeten Gruppe enthält.

**21.** Verfahren nach einem der vorhergehenden Ansprüche, dadurch gekennzeichnet, daß das erhaltene Material den Katalysatorträger bildet, wobei der Katalysator ferner wenigstens ein Element, ausgewählt aus der durch Titan und Bor gebildeten Gruppe enthält.

**22.** Verfahren nach einem der vorhergehenden Ansprüche, dadurch gekennzeichnet, daß die Charge eine $C_5$-Olefin-

charge ist.

**23.** Verfahren nach einem der vorhergehenden Ansprüche, dadurch gekennzeichnet, daß das molare Verhältnis Wasser/olefinische Kohlenwasserstoffe zwischen 0,1 und 3 liegt.

**24.** Verfahren nach Anspruch 22, dadurch gekennzeichnet, daß es in Abwesenheit von Wasser abläuft.